(19) Europäisches Patentamt
European Patent Office
Office européen des brevets

(11) **EP 2 886 655 A1**

(12) **EUROPEAN PATENT APPLICATION**

(43) Date of publication:
**24.06.2015 Bulletin 2015/26**

(51) Int Cl.:
*C12P 7/08* (2006.01)   *C12P 5/02* (2006.01)
*C12P 3/00* (2006.01)   *C02F 11/04* (2006.01)
*B01J 27/24* (2006.01)   *B01J 31/06* (2006.01)

(21) Application number: **13198792.7**

(22) Date of filing: **20.12.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Methodo Chemicals S.r.l.**
**42017 Novellara (IT)**

(72) Inventor: **Beltrami, Andrea**
**42017 Novellara (IT)**

(74) Representative: **Villa, Livia et al**
**Notarbartolo & Gervasi S.p.A.**
**Corso di Porta Vittoria, 9**
**20122 Milano (IT)**

(54) **Use of a polyamine in processes for producing biofuels by anaerobic digestion of organic biomasses**

(57)    The use of polyamine in processes for producing biofuels by means of anaerobic digestion of organic biomasses is described. In particular, the presence of polyamine allows to increase the action of hydrolytic enzymes, facilitating and promoting the process of hydrolysis and saccharification, but especially increasing the final yields in biofuels by reduction of the output non-digested biomass.

EP 2 886 655 A1

**Description**

FIELD OF THE INVENTION

The present invention relates to the use of a polyamine in processes for producing biofuels by anaerobic digestion of organic biomasses. In particular, the presence of a polyamine allows to increase the action of hydrolytic enzymes, while facilitating and promoting the process of hydrolysis and saccharification, but especially increasing the final yields in biofuels by reduction of the output non-digested biomass.

STATE OF THE ART

Recently, the agricultural and zootechnical production line was directly affected by energy productions: renewable energies and green economy have driven the agricultural world to producing electrical energy or biofuels from renewable sources through different forms of incentive.

Renewable energy plants of the agricultural type, excluding photovoltaic ones, in particular are for producing methane and for producing combustible ethanol. Both of these processes have as a common factor the anaerobic digestion of organic biomasses of various kinds: food waste, organic waste, zootechnical slurry, but also agricultural raw materials (agricultural biomasses) and animal feed.

The anaerobic digestion consists in a complex and non-standardized (from neither the chemical-physical nor the microbiological point of view) microbiological process which envisages the conversion of complex organic polymers (sugars, starches, fibres) to organic acids, hydrogen and carbon dioxide which, depending on the microbiological typing, are in turn converted to the so called biofuels (methane and ethanol); the cause of the lack of standardization is due to the variety of plant types and of selection of plant conduction which, therefore, affects the development of variable microbiological floras.

One of the main problems in this type of plants is the low degradability of the components more resistant to fermentations, particularly fibrous and structural cellulose-based components. The cause of the resistance may be due to a poor microbiological efficiency, to the size of the plant (and therefore of its hydraulic flows) or the type of matrix (higher or lower presence of lignin, for example).

The object of the present invention is therefore to improve the efficiency of such processes, while increasing at the same time the yields of the final biofuel.

SUMMARY OF THE INVENTION

The object indicated above has been achieved by a process for producing biofuel by anaerobic digestion of biomass in the presence of at least one hydrolytic enzyme and at least one polyamine, as per claim 1.

For the purposes of the present invention, the term 'biofuel' means biogas, biomethane, bioethanol, hydrogen, or a mixture thereof.

The term 'biomass' means cellulose or pastes thereof, sludges, or treatment water, molasses, starches or flours from crops, silage crops, crops in general (also not in silage) or in the form of animal feed or pellets/flakes, slurry, exhausted zootechnic litters and manure containing crude cellulose, sewage and waste waters, non-treated or pre-treated sludge, sludge and/or organic fractions of urban solid waste and/or waste products or by-products of the food industry or transformation of agricultural products or animals, and mixtures thereof.

The term 'hydrolytic enzyme' means xilanase, amylase, glucanase, cellulase, or a mixture thereof, isolated and purified or supported on plant material or inert mineral, coming from industrial biotechnological GMO and non-GMO productions, or from specific microorganisms cultured on dedicated liquid or solid substrates, such as bacteria, fungi, or yeasts.

'Polyamine' means a polymer having 1 to 15 amino groups, preferably 1 to 10 amino groups, more preferably 3 to 8 amino groups. Examples of polyamines include chitosan, ethylenediamine, diethylenetriamine, triethylenetetramine, tetraethylenepentamine, pentaethylenehexamine, dipropylenetriamine, tripropylenetetramine, tetrapropylenepentamine, pentapropylenehexamine, polyethylenimine having an average degree of polymerization (corresponding to the number of nitrogen atoms) of, for example, 10, 35, 50 o 100, and also polyamines that have been obtained by reacting oligoamines (with chain extension) with acrylonitrile and subsequent hydrogenation, for example N,N'-bis-(3-aminopropyl)ethylenediamine.

According to another aspect, the invention relates to a composition consisting of at least one hydrolytic enzyme and at least one polyamine.

The features and the advantages of the present invention will be apparent from the detailed description reported below.

DETAILED DESCRIPTION OF THE INVENTION

The invention therefore concerns a process for producing biofuel comprising the steps of:

    i) feeding biomass to an anaerobic digester,
    ii) feeding digestate comprising at least one hydrolytic enzyme,
    iii) adding at least one polyamine,
    iv) allowing the resulting mass to react in anaerobic conditions, and
    v) separating the biofuel obtained.

In fact, it has been surprisingly observed that the presence of at least one polyamine allows to advantageously increase the efficiency and yield of final biofuel.

Digestate is the fermenting means of the process of the invention and is obtained as a process by-product of anaerobic digestion. Digestate is a material that, as compared to the starting biomasses, is homogeneous, with a higher humidity rate because part of the dry matter has been degraded biologically, i.e. degraded by bacteria. The remaining organic matter is more stable and contains elements of fertility, such as nitrogen, phosphorus and potassium.

The efficiency of anaerobic digestion of the process of the invention is determined by the effective yield in biofuel as compared to the theoretical yield deriving from the starting biomass.

The combined use of at least one hydrolytic enzyme and at least one polyamine surprisingly increases the hydrolysis of the complex polymers present in the biomass, such as starches, cellulose, glucanes, xilanes, hemicelluloses, proteins, in simpler compounds, such as sugars, organic acids, amino acids.

For industrial plants having structural, conduction or management problems, the yields may be disadvantageously lower than expected; this happens especially when the biomass turnover within the process of anaerobic digestion is very rapid. On the contrary, the process of the invention also advantageously addresses this problem by offering higher yields owing to the presence of at least one polyamine. This adds to the further advantage that the addition of said at least one polyamine does not require dedicated facilities or equipment, nor particular modifications to pre-existing biofuel production plants.

Furthermore, the use of said at least one polyamine does not cause the formation of substances considered detrimental to the environment and, as being biodegradable, the waste products coming from the anaerobic digester, in turn, do not show risk indicators for the health or the environment.

The process of the invention may be both a continuous and a batch process, both with complete mix digesters and plug-flow digesters. The temperatures are variable from RT (= no heating) to 60°C (in the art, the plants are said to be operating in psychrophyly, mesophyly and thermophyly). The dry matter may be from about 1 % to 50% by weight on the weight of the biomass.

Preferably, steps i)-iii) are carried out simultaneously or substantially simultaneously.

Preferably, said at least one polyamine is at a concentration of 50-1000 ppm in the anaerobic digester.

Such a concentration can be calculated and verified by known methods, such as the measurement of viscosity, as per Huggins and Kraemer equations (Rosen S.L. *Fundamental principles of polymeric materials* 2nd Ed.: John Wiley and Sons: New York. 1993: 58-68).

More preferably, said at least one polyamine is at a concentration of 50-500 ppm in the anaerobic digester.

Even more preferably, said at least one polyamine is at a concentration of 50-250 ppm in the anaerobic digester.

In preferred embodiments, said at least one polyamine has a molecular weight of 1000-8000 Da, a viscosity (Brookfield) of 150-200 cps (5 g/l), 70-100 cps (2.5 g/l), and 25-50 cps (1 g/l), and a bulk density of 0.50-0.95 g/cm$^3$.

In a particularly preferred embodiment, said at least one polyamine has a molecular weight of 1000-8000 Da, a viscosity (Brookfield) of 180 cps (5 g/l), 85 cps (2.5 g/l), and 35 cps (1 g/l), and a bulk density of 0.85 g/cm$^3$.

Optionally, one or more hydrolytic enzymes may be added to the anaerobic digester, if the digestate itself contains them in an insufficient amount or in order to increase the effectiveness of the process.

Generally, the greater the amount of enzyme, the greater is the fermentation activity and thus the final production of biogas. Therefore, in order to add a further enzyme to the digester, it may be envisaged an additional amount of at least 10 g/ton of biomass fed to the digester.

In a preferred embodiment, said at least one polyamine and said one or more additional hydrolytic enzymes are in the form of an adduct or are chemically bound to one another, therefore in step ii) also the addition of one or more hydrolytic enzymes is simultaneously carried out.

In another embodiment, in step iii) also cationic or anionic polyacrylamide, carboxymethylcellulose, or a mixture thereof, is further added. Preferably, said polyacrylamide has a molecular weight of 6-10 MDa.

More preferably, said cationic or anionic polyacrylamide, carboxymethylcellulose, or a mixture thereof, is at a concentration of 50-1000 ppm in the anaerobic digester. The measurement of such concentration may be assessed as reported above for the at least one polyamine.

Similarly, also said cationic or anionic polyacrylamide, carboxymethylcellulose, or a mixture thereof, and said at least one hydrolytic enzyme may be in the form of an adduct or chemically bound to one another.

According to another aspect, the present invention relates to a composition consisting of at least one hydrolytic enzyme, at least one polyamine and/or cationic or anionic polyacrylamide, or a mixture thereof.

In a preferred embodiment of the composition, said at least one hydrolytic enzyme, said at least one polyamine and/or said cationic or anionic polyacrylamide, or a mixture thereof, are in the form of an adduct or are chemically bound to one another.

Preferably, said composition is a solid form, more preferably in the form of powder, beads, flakes, capsules, sticks, bars, pellets, multiparticulate matter, micronized particulate matter, agglomerates, micro-granules, granules, or tablets. Alternatively, said composition is in the form of a liquid, gel, suspension or emulsion.

Optionally, said composition may be coated by means of a coating or microencapsulated in order to obtain an extended release formulation.

In order to test the effectiveness of the process of the invention, a comparison test was carried out between a process of methanation in the absence of polyamine (control - C) and a process of methanation in the presence of a polyamine, in particular at a concentration of about 100 ppm (test - T), according to the invention.

The reference protocol selected was the UNI EN ISO 11734:2004 standard adapted to carry out the test with the addition of an agricultural raw material to the fermentation medium, that is corn silage.

A mixture of organic biomass and fresh digestate, proportioned according to the requirement of the standard at section 6.3, in order to reach 100 mg/L of organic carbon (OC), is incubated at 38°C $\pm$ 2°C in hermetically closed containers for a period of about 60 days.

In the test container, the increase in the head space pressure, due to the generation of carbon dioxide ($CO_2$) and methane ($CH_4$) was determined quantitatively. The pattern of biodegradation may be followed by carrying out measurements at intermediate times of the sole gas production. In order to have additional information, it is possible to determine primary biodegradation at the beginning and at the end of the test, by means of specific analyses on the starting and final fermentation medium.

The thus adapted test reflects fermentation systems entirely similar to industrial ones for the production of biofuels.

The stock solution is the basic medium wherein the other components are to be added. In particular, as indicated in the standard above, two stock solutions were made:

o Control Solution, as a standard dilution solution such that, at preset amounts, it contains the following constituents.

Anhydrous potassium dihydrogen phosphate ($KH_2PO_4$) 0.27 g
Dodecahydrate disodium phosphate ($Na_2HPO_4 \cdot 12H_2O$) 1.12 g
Ammonium chloride ($NH_4Cl$) 0.53 g
Dihydrate calcium chloride ($CaCl_2 \cdot 2H_2O$) 0.075 g
Hexahydrate magnesium chloride ($MgCl_2 \cdot 6H_2O$) 0.10 g
Tetrahydrate iron chloride (II) ($FeCl_2 \cdot 4H_2O$) 0.02 g
Resazurin (oxygen indicator) 0.001 g
Nonahydrate sodium sulfide ($Na_2S \cdot 9H_2O$) 0.1 g

o TEST Solution, envisages the same composition of the Control Solution with the addition of polyamines in order to obtain an emulsion. The amount of polyamines added to the fermentation medium was such that at the final volume, the polyamines were at a concentration of about 100 ppm.

De-oxygenated water (containing less than 2 mg/l of dissolved organic carbon (DOC) quantum satis to reach a volume of 1 L. In order to achieve anoxic conditions, it is bubbles in the medium, immediately before its use, of nitrogen for about 20 min, so as to eliminate oxygen. The pH of the medium is adjusted with mineral acid or base diluted, if necessary, up to 7 $\pm$ 0.2.

Polyamines

The polyamines used have a molecular weight of 1000-8000 Da, a viscosity (Brookfield) of 180 cps (5 g/l), 85 cps (2.5 g/l), and 35 cps (1 g/l), and a bulk density of 0.85 g/cm$^3$.

Digestate

A sufficient amount of digestate is provided coming from a plant powered in a way that is as similar as possible to the organic biomass fed to the test (example: a plant powered at 70% of corn silage and 30% of slurry if a biomass mixture of fresh zootechnical effluents and corn silage is to be tested).

Biomass

The biomass may be in the form of a stock solution, a suspension, an emulsion, or directly as a solid or liquid, so that it produces a test concentration of 100 mg/l of organic carbon. For test compounds that are not sufficiently water-soluble, reference can be made to the ISO 10634 standard, but without using organic compounds such as chloroform or carbon tetrachloride which, as is know, inhibit the production of methane.

Inoculum

Right before use, wash the digestate so as to reduce the concentration of Inorganic Carbon in the test solution at less than 10 mg/l, initially spinning in test tubes, hermetically closed, at a relatively low speed (for example 3,000 g), for a maximum time of 5 min. Suspend in the dilution medium, oxygen-free, the bead precipitate, spin and eliminate washes. If the IC value has not lowered sufficiently, was the sludge two more times at utmost. Lastly, suspend the precipitate in the required volume of the dilution medium and determine the concentration of the total solid content. The final concentration of the total solid content should be in range of 1 g/l to 3 g/l. Perform these operations so that the contacting of the sludge with oxygen is reduced to the minimum (for example operating under nitrogen atmosphere). A Control inoculum, produced with the Control Solution, and a Test inoculum, produced with the TEST Solution, will be thus obtained. Incubation or water-bath equipment, or sand, are thermally controlled at 35°C $\pm$ 2°C.
A needle pressure measurement device is also used, for example a manometer connected to a suitable syringe needle; a gas-tight three way valve facilitare the release of excess pressure. The device must be used and calibrated based on the manufacturer's instructions. It is necessary to maintain, to the lowest value possible, the inner volume of the valve and the piping of the pressure transducer, so that any errors introduced by disregarding the volume of the equipment are negligible.
A carbon analyzer is further used, adapted to directly determine organic carbon in the range of 1 mg/l to 200 mg/l.

METHOD OF COMPARISON

Carry out the following starting procedures, using techniques suitable for maintaining the contact between digested sludge and oxygen to the lowest values possible, for example operating in an anaerobic chamber under nitrogen atmosphere, or purging the bottles with nitrogen.

*Preparation of test samples and control samples*

Mix in appropriate test containers the inoculum, about 100 g of test biomass and digestate. The final test concentration of carbon usually must be of 100 mg/l, therefore, the amount of biomass will be weighted based on this parameter. At the end of the preparation, two final mixtures will be obtained, one produced with the base inoculum (Control) and one produced with an inoculum including polyamines at the concentration of about 100 ppm (TEST).
Make sure that the total liquid volume (or initial Volume, Vi) and the one of the head Volume (Vh) are the same in all the containers. Note down Vi and Vh. If necessary, add anoxic test medium. Hermetically close each of the containers with the gas-impermeable partition and incubate at 38°C. A regular mixing of incubated bottles is advisable. Observe the containers after an incubation time of 24 h to 48 h. Discard those containers wherein the content has a clear pink color of the supernatant, that is if resazurin has shown color variation, indicating the presence of oxygen. While small amounts of oxygen may be tolerated by the system, higher concentrations may significantly inhibit the development of the anaerobic biodegradation. Mix the content of each container by stirring, or shaking for a few minutes at least one or two times per week, and before each pressure measurement. Measure gas pressure, for example passing the syringe needle, connected to the pressure monitoring device, through the partition. Record pressure in millibars. Stirring brings the inoculum back to the suspension and ensures gas balance. While measuring the pressure, the gas in the head space must be kept to the same temperature as that of digestion. Attention should be paid to prevent water from entering the syringe needle. In that case, dry any wet parts and insert a new needle. In order to obtain the value of gas pressure, measure the pressure within the containers once a week, release the excess gas in the atmosphere or only measure the pressure at the end of the test, in order to determine the amount of biogas produced.
Complete the test after a period of incubation of 60 days, unless the biodegradation curve obtained from the pressure measurement has not reached the plateau step, this is the step wherein the maximum degradation was reached and it indicates a sufficient degree of biodegradation (>50%) to complete the test. If, at the end of the usual period of incubation, the plateau step has not been clearly reached, the test should be continued until reaching such step.

ANALYSIS OF RESULTS

Take a sample of the final mixture at the beginning of the test and at the end of the test (one digestion has completed).

Measure total solids (SS%) by stove drying, and volatile solids (SV%) by incineration in muffle furnace.

BIOGAS PRODUCTIONS measured by normal cubic metres of biogas per ton of volatile solids dissolved into the fermentation medium:

CONTROL = 592.2 $Nm^3/t_{SV}$

TEST = 629.1 $Nm^3/t_{SV}$

DEGREE OF BIODEGRADATION: MAXIMUM ANAEROBIC DEGRADABILITY measured as a percentage of volatile solids converted into the fermentation medium:

CONTROL = 75.7%

TEST = 81.4%

50% DEGRADABILITY TIMES, measured as the time needed to degrade 50% of volatile solids globally converted into gas:

CONTROL = 6.4 days

TEST = 5.9 days

The measurement of the volumes of biogas produced shows that in the TEST sample an increase of biogas production by about 6%, an increase in overall degradability of organic matter by 5.7% and a reduction in degradability times by about 12 hours.

In the hypothesis that the ashes are not converted to biogas, from the analysis of the digestate the phenomenon of concentration of solids into the fermentation medium by biogas release is considered and therefore, the following is obtained:

$$Concentration\ Factor\ of\ medium = \frac{Ashes(OUT)}{Ashes(IN)}$$

$$\%\ PROCESS\ EFF. = \left[1 - \left(\frac{\left\lfloor\frac{SV(OUT)}{(Conc.\ Fatt.)}\right\rfloor}{SV(IN)}\right)\right] * 100$$

CONTROL (C) test process efficiency:

| IN | | |
|---|---|---|
| ST [g/kg] | SV [g/kg] | Ashes [g/kg] |
| 49.94 | 37.66 | 12.28 |
| OUT | | |
| ST [g/kg] | SV [g/kg] | Ashes [g/kg] |
| 37.60 | 25.91 | 11.69 |
| Process efficiency (C) = 27.73% | | |

TEST (T) test process efficiency:

| IN | | |
|---|---|---|
| ST [g/kg] | SV [g/kg] | Ashes [g/kg] |
| 63.27 | 49.81 | 13.46 |
| OUT | | |
| ST [g/kg] | SV [g/kg] | Ashes [g/kg] |
| 42.30 | 29.30 | 13.00 |
| Process efficiency (T) = 39.10% | | |

The above-reported test shows how the process of the invention in the presence of polyamines at the concentration of about 100 ppm has increased its hydrolytic efficiency by more than 11 % with an increase in biogas production by about 6% as compared to the control process. The increase in the process efficiency is considered to be significant and capable of widely accounting for the cost deriving from the use of polyamines.

Based on the results obtained, the possibility of using polyamines in anaerobic fermentations is advantageous especially in those cases in which the output waste products (sludge or digestates) still have a share of "non-digested", typically cellulose-based, organic matter.

**Claims**

1. A process for the production of a biofuel comprising the steps of:

   i) feeding biomass to an anaerobic digester,
   ii) feeding digestate comprising at least one hydrolytic enzyme,
   iii) adding at least one polyamine,
   iv) allowing the resulting mass to react in anaerobic conditions, and
   v) separating the biofuel obtained.

2. The process of claim 1, wherein said at least one polyamine is at a concentration of 50-1000 ppm in the anaerobic digester.

3. The process of claim 1 or 2, wherein said at least one polyamine is at a concentration of 50-500 ppm in the anaerobic digester.

4. The process of claim 3, wherein said at least one polyamine has a molecular weight of 1000-8000 Da, a viscosity (Brookfield) of 150-200 cps (5 g/l), 70-100 cps (2.5 g/l), and 25-50 cps (1 g/l), and a bulk density of 0.50-0.95 g/cm$^3$.

5. The process of any one of claims 1-4, wherein one or more hydrolytic enzymes are further added to the biomass.

6. The process of claim 5, wherein said at least one polyamine and said one or more additional hydrolytic enzymes are in the form of an adduct or are chemically bound to one another.

7. The process of any one of claims 1-6, wherein said at least one polyamine is selected from chitosan, ethylenediamine, diethylenetriamine, triethylenetetraamine, tetraethylenepentamine, pentaethylenehexamine, dipropylenetriamine, tripropylenetetramine, tetrapropylenepentamine, pentapropylenehexamine, and polyethyleneimine.

8. The process of any one of claims 1-7, wherein said hydrolytic enzyme is xinalase, amylase, glucanase, cellulase or a mixture thereof.

9. The process of any one of claims 1-8, wherein in step iii) also cationic or anionic polyacrylamide, carboxymethylcellulose or a mixture thereof is further added.

10. The process of claim 9, wherein said cationic or anionic polyacrylamide, carboxymethylcellulose or a mixture thereof is at a concentration of 50-1000 ppm in the anaerobic digester.

11. The process of claim 9 or 10, wherein said cationic or anionic polyacrylamide, carboxymethylcellulose or a mixture thereof and said one or more additional hydrolytic enzymes are in the form of an adduct or are chemically bound to one another.

12. A composition consisting of at least one hydrolytic enzyme, at least one polyamine and/or cationic or anionic polyacrylamide, carboxymethylcellulose, or a mixture thereof.

13. The composition of claim 12, wherein said at least one hydrolytic enzyme, said at least one polyamine and/or said cationic or anionic polyacrylamide, or a mixture thereof, are in the form of an adduct or are chemically bound to one another.

14. The composition of claim 12 or 13, said composition being in the solid form selected from powder, beads, flakes, capsules, sticks, bars, pellets, multiparticulate matter, micronized particulate matter, agglomerates, microgranules, granules or tablets.

15. The composition of claim 12 or 13, said composition being in the form of a liquid, gel, suspension or emulsion.

# EUROPEAN SEARCH REPORT

**Application Number**

EP 13 19 8792

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KHEMKHAO ET AL: "Effect of chitosan on UASB treating POME during a transition from mesophilic to thermophilic conditions", BIORESOURCE TECHNOLOGY, vol. 102, 2011, pages 4674-4681, XP028154110, * See pages 4674-4675 (Abstract + Introduction) and pages 4680-4681 * | 1-15 | INV. C12P7/08 C12P5/02 C12P3/00 C02F11/04 B01J27/24 B01J31/06 |
| A | BASILE ET AL: "The effect of the surface charge of hydrogel supports on thermophilic biohydrogen production", BIORESOURCE TECHNOLOGY, vol. 101, 2010, pages 4386-4394, XP026953074, * See page 4392 (Figure 3b; H2, GCH) * | 1-15 | |
| A | LAKANIEMI ET AL: "Biogenic hydrogen and methane production from Chlorella vulgaris and Dunaliella tertiolecta biomass", BIOTECHNOLOGY FOR BIOFUELS, vol. 4, 2011, pages 34(1)-45(12), XP021111198, * See page 37(4), Table 1 (chitosan) and page 42(9), left column [8] * | 1-15 | TECHNICAL FIELDS SEARCHED (IPC) C12P C02F B01J |
| A | CARVER ET AL: "Thermophilic, anaerobic co-digestion of microalgal biomass and cellulose for H2 production", BIODEGRADATION, vol. 22, 2011, pages 805-814, XP019910444, * See page 807 (chitosan, flocculation) * | 1-15 | |

-/--

The present search report has been drawn up for all claims

| Place of search | Date of completion of the search | Examiner |
|---|---|---|
| Berlin | 13 May 2014 | Korsner, Sven-Erik |

EPO FORM 1503 03.82 (P04C01)

Europäisches
Patentamt

European
Patent Office

Office européen
des brevets

**EUROPEAN SEARCH REPORT**

Application Number

EP 13 19 8792

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NO ET AL: "Application of chitosan for treatment of wastewaters", REVIEWS OF ENVIRONMENTAL CONTAMINATION AND TOXICOLOGY, vol. 163, 2000, pages 1-28, XP001525071, * See pages 1-2 * | 1-15 | |

----

TECHNICAL FIELDS
SEARCHED        (IPC)

The present search report has been drawn up for all claims

| Place of search | Date of completion of the search | Examiner |
|---|---|---|
| Berlin | 13 May 2014 | Korsner, Sven-Erik |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)